Europäisches Patentamt

European Patent Office

Office européen des brevets

(11) Publication number: **0 543 898 B1**

# EUROPEAN PATENT SPECIFICATION

(45) Date of publication of patent specification: **07.09.94**

(51) Int. Cl.5: **C01B 33/34**, B01J 29/04, C07C 5/41, C07B 35/02

(21) Application number: **91915014.4**

(22) Date of filing: **14.08.91**

(86) International application number:
**PCT/EP91/01547**

(87) International publication number:
**WO 92/03379 (05.03.92 92/06)**

(54) **ZINC-CONTAINING ZEOLITE L.**

(30) Priority: **16.08.90 GB 9018003**

(43) Date of publication of application:
**02.06.93 Bulletin 93/22**

(45) Publication of the grant of the patent:
**07.09.94 Bulletin 94/36**

(84) Designated Contracting States:
**AT BE CH DE DK ES FR GB GR IT LI LU NL SE**

(56) References cited:
**EP-A- 0 219 354**

**CHEMICAL ABSTRACTS, vol. 78, no. 6, 12 March 1973, Columbus, Ohio, US; abstract no. 34714M, CRUECEANU M. AND AL.: 'ZEOLITES SYNTHESES IN THE PRESENCE OF SOME MINERAL SALTS' page 323; column L; & AN. STIINT. UNIV. "AL. I CUZA" IASI, SECT. 1C see abstract**

(73) Proprietor: **EXXON CHEMICAL PATENTS INC.
200 Park Avenue
Florham Park New Jersey 07932 (US)**

(72) Inventor: **VERDUIJN, Johannes, Petrus
Westersingel 34
NL-3202 XL Spijkenisse (NL)**

(74) Representative: **Bawden, Peter Charles et al
EXXON CHEMICAL LIMITED
EXXON CHEMICAL TECHNOLOGY CENTRE
PO Box 1
Abingdon Oxfordshire OX13 6BB (GB)**

**Description**

The present invention relates to a zeolite L containing non-exchangeable zinc, and a process for its production.

Zeolites (molecular sieves based on tetrahedra of oxides of silicon and aluminium) are well known as catalysts for a variety of organic reactions. For example, they may be used to catalyse the aromatisation of alkanes.

Metal ions such as zinc, magnesium, cobalt or nickel may be ion exchanged with a zeolite. Examples of such zeolites are described in US-A-3729515, US-A-4172843 and EP-A-00711316. The metals which enter the zeolite through ion exchange may also be removed from the zeolite by ion exchange.

Zeolite L, such as zeolite KL may be used as a catalyst in aromatisation reactions. The catalytic properties may be improved by increasing the electron donor properties of the zeolite, by reducing the overall electronegativity. Reduced electronegativity can be achieved by altering the chemical composition of the zeolite. For example, the ratio of Si/Al may be reduced; the potassium may be partly replaced by a larger alkali metal such as caesium; the aluminium may be partially replaced by other metals.

EP-A-219354 describes a method of suppressing zeolite W formation in the preparation of zeolite L by introducing small amounts of a metal into the synthesis gel. Typically less than 0.1 wt% metal is introduced.

The present invention provides a zeolite L comprising silicon, aluminium and 1 to 10% by weight of non-exchangeable zinc.

The present zeolite shows under analysis by X-ray diffraction and thermographic methods that this zeolite containing non-exchangeable zinc has the structure of zeolite L. Zeolite L crystals can be, for example, cylindrical in shape. The term "cylindrical" is used herein to describe the shape of a cylinder as defined in solid geometry - that is, a solid bounded by a surface generated by a line moving parallel to a fixed line so as to cut a fixed plane curve and bounded by two parallel planes (basal planes) which cut the surface.

The amount of zinc in the zeolite can be selected depending on the desired end use. Up to 10 weight percent may be present, typically 2 to 7% by weight. The greater the weight percent of Zn the lower the weight percent of Al tends to be, as the Al is replaced in the zeolite structure with Zn.

The $Zn/Al_2$ atomic ratio in the zeolite is generally from 0.3 to 3.5. For ease of synthesis it is preferred to use a molar ratio of preferably 0.5 to 2.0, more preferably from 0.9 to 1.5, although ratios outside these preferred ranges may be used if the desired end use suggests that this would be desirable. If the level of zinc is too low, the resulting reduction in electronegativity in the zeolite tends to be insignificant. If too much zinc is present, the crystal formation rate is low and it becomes difficult to form a crystalline product with the zeolite-L structure. If the level of zinc in the synthesis mixture is too high the basal planes of the resulting zeolite crystals become very rough. This is undesirable in e.g. an aromatisation catalyst.

It is believed that the zinc is present in the zeolite in the form of ZnO. The zeolite may be a zeolite KL. In this case, typical molar ratios in the zeolite in anhydrous form are $K_2O/0.1\text{-}0.6$ $ZnO/0.4\text{-}0.9$ $Al_2O_3/4\text{-}6$ $SiO_2$. Part of the potassium may be replaced by other alkali metals such as sodium.

The molar ratio of $K_2O/(Al_2O_3 + ZnO)$ can be used to indicate whether the zinc forms part of the zeolite framework. This ratio should be in the region of 1 if the zinc is part of the framework. Generally it is found experimentally to be in the region of 0.85-1.15, typically 0.89 to 1.00.

The zeolites of the present invention may be produced in a manner similar to the production of conventional zeolites-L. In particular, a synthesis mixture comprising a source of ZnO, a source of $Al_2O_3$, a source of $SiO_2$ and water is heated, typically at a temperature of 140 to 200 °C. The system may be seeded with a small quantity of preformed zeolite crystals. The smaller the crystals are, the more effective the seeding and on a weight basis less seeding material is required.

If the zeolite is a zeolite KL then the synthesis mixture also comprises a source of $K_2O$, and the seed crystals comprise KL-seed crystals.

The source of ZnO may be any Zn source which is soluble in alkaline solution e.g. a zincate solution such as zinc nitrate or zinc acetate. Typically the source of $Al_2O_3$ is provided as an aluminate solution. When producing a zeolite KL the synthesis mixture can conveniently be produced by providing the sources of ZnO, $Al_2O_3$ and $K_2O$ as a potassium aluminate/zincate solution, by forming an aqueous solution of potassium, aluminium and zinc ions e.g. forming a solution of potassium hydroxide, aluminium hydroxide and zinc nitrate.

The source of $SiO_2$ may be, for example, silica, preferably in a colloidal form such as is available commercially.

The ingredients of the synthesis mixture are mixed together to form a gel. The synthesis mixture before heating will generally have a composition of 2.5-4.8 $K_2O/xZnO/(1\text{-}x)Al_2O_3/9\text{-}14$ $SiO_2/150\text{-}220$ $H_2O$ where x is

0.1 to 0.75, preferably 0.3 to 0.6.

Small amounts of an additional metal may also be added to the synthesis mixture, as is described in EP-A-0219354. This may be used to control production of the desired zeolite and suppress unwanted forms of zeolite, such as zeolite W, which may otherwise result from a particular synthesis mixture. The additional metal is preferably an alkaline earth metal such as magnesium, calcium or barium, or a transition metal such as manganese, chromium, cobalt or nickel.

The molar ratio of the additional metal M may be as follows:

$(K_2O + M_{2/n}O)/SiO_2$ is 0.18 to 0.36

$H_2O/(K_2O + M_{2/n}O)$ is 25 to 90

$K_2O/(M_{2/n}O)$ is 0.9 to 0.999.

where n is the valency of the additional metal, M, and part of the potassium may be replaced by another alkali metal. Amounts of M used in the synthesis mixture, may be as little as 0.1 to 1 ppm or may be as much as 0.1 wt %. If M is present then preferably it constitutes 5 ppm to 0.05 wt % of the synthesis mixture.

The synthesis mixture is typically aged at 140 to 200°C, preferably 150 to 180°C, under quiescent conditions i.e. without stirring.

Generally the heating step to allow crystallisation to occur lasts at least 40 hours depending on the amount of zinc. Heating times may be, for example 40 to 200 hours, such as 40 to 100 hours. A synthesis mixture with a greater $Zn/Al_2$ atomic ratio will generally require a longer heating time to reach the same level of crystallisation as a corresponding mixture containing a lower $Zn/Al_2$ atomic ratio at a given crystallisation temperature. In other words the greater the level of zinc in the mixture the longer it takes to produce the zeolite at a given crystallisation temperature. Spot samples may be taken at intervals during heating and examined e.g. by X-ray diffraction to establish the level of crystallisation reached. It is believed that such sampling does not significantly affect the results of the crystallisation process.

Once the heating is completed i.e. the mixture has reached the required level of crystallisation , the product may be washed and dried before being used in catalysis.

The zeolites of the present invention have been found to have toluene adsorption capacities which are comparable to those of corresponding alumino-zeolites L. The zeolite crystals have a diameter (in micrometers) of 0.3 to 0.6 for example 0.4 to 0.5, and have a crystal length (in micrometers) of 0.07 to 0.5 for example 0.1 to 0.4.

It is believed that the zeolites of the present invention may be used in the same way as corresponding alumino-zeolites KL. For example they may be extruded and used in the following processes:

- selective dehydrogenation of light alkanes into the corresponding alkenes,
- cyclisation catalysis,
- selective hydrogenation of aromatics into cyclic alkenes,
- selective adsorption of S-compounds,
- dehydrogenation of ethyl-benzene to styrene/cyclohexanal to cyclohexane.

For example, benzene may be selectively hydrogenised to cyclohexene. Attempts to carry out this process had in the past used e.g. a catalyst comprising 1 to 3% ruthenium on a support. However, it was necessary to carry out the hydrogenation in water containing up to 4M alkaline and zinc ions in order to obtain cyclohexene selectivity. In this system the catalyst stability is poor and the use of large quantities of alkaline and zinc ions is undesirable.

Zeolite L and particularly the zeolites of the present invention are suitable for use as catalytic supports for ruthenium in this process, and achieve an acceptable selectivity and yield, without the need for the addition of alkaline or zinc ions.

Similar zeolites may also be used in the selective hydrogenation of benzene to cyclohexene.

The following examples illustrate the invention.

EP 0 543 898 B1

EXAMPLE 1

Synthesis mixture with $Zn/Al_2$ atomic ratio of 0.50.

| Potassium aluminate/zincate solution (wt of reactants in grams): | |
|---|---|
| KOH pellets (86.5 % purity) | 44.01 |
| Al(OH)$_3$ (98.6 % purity) | 7.92 |
| Zn(NO$_3$)$_2$6H$_2$O (> 98 % purity) | 7.44 |
| H$_2$O | 73.60 |

The solution was boiled until clear, cooled to 50°C and corrected for water loss due to boiling.

| Silicate Solution: | |
|---|---|
| Ludox H5-40 (DuPont) | 150.27 |
| KL-seed crystals | 0.82 |
| H$_2$O | 115. 59 |

The seed crystals were dispersed in a portion of the water using an ultrasonic bath. The dispersed seeds were added to the Ludox.

The two solutions were mixed together in a household mixer for 5 minutes. The molar composition of the synthesis mixture was:

3.40 $K_2O$/0.25 ZnO/0.50 $Al_2O_3$/10 $SiO_2$/161 $H_2O$ + 0.20 wt % KL seeds (based on gel weight).

329.25 g of the synthesis mixture was transferred to a 300 ml stainless steel autoclave and aged at autogeneous pressure during 48 hours at 150°C without stirring. The resulting product was washed with demineralized water to pH 10.0 and dried for 16 hours at 150°C. The weight of the recovered product was 33.0 g. This corresponds to a product yield of 10.0 % (wt 150°C dry product/wt of gel x 100%).

A similar synthesis mixture not containing zinc and crystallized under the same conditions invariably gave a product yield of 7.0 + 0.2 %. The significant increase in yield for the Zn-containing synthesis mixture suggested that the product contained zinc.

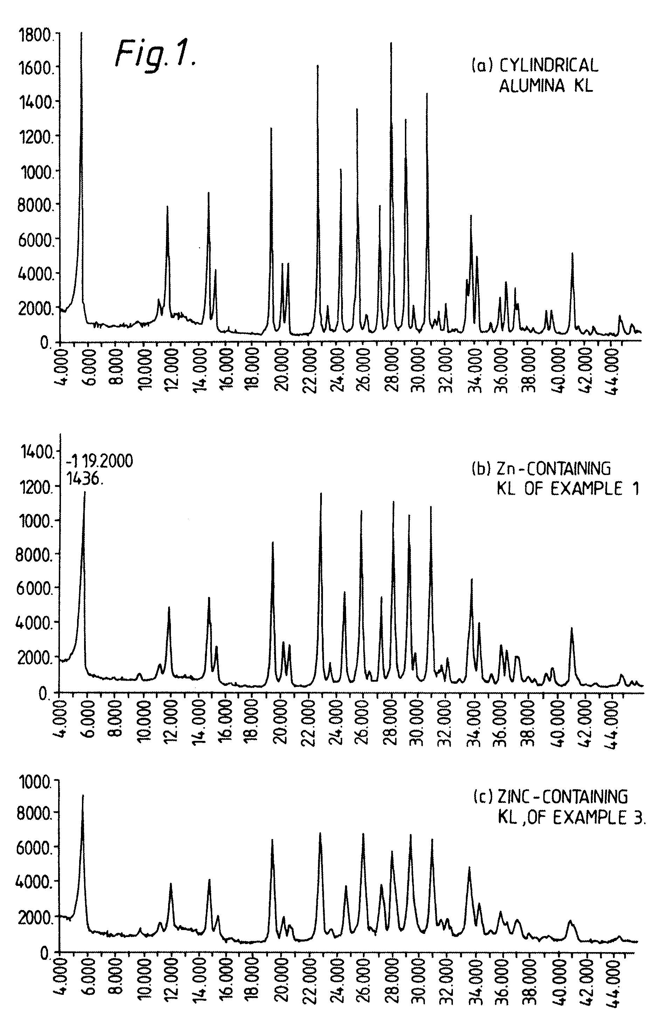

X-Ray diffraction (XRD) showed that the product was excellently crystalline and pure, e.g. no peaks other than those of zeolite-L could be seen. Comparative diffractograms of conventional alumino zeolite KL and the Zinc-containing alumino-KL are shown in Figures 1(a) and (b). The same peaks due to the zeolite structure can be seen in both diffractograms.

The toluene adsorption capacity was 10.5 wt % at P/Po = 0.25 and T = 30°C. For comparison, the toluene adsorption capacity of "conventional" zeolite-L is between 9.5 and 10.5. Scanning electron microscopy (SEM) micrographs of the product showed that the product consisted of disk-type crystals with a diameter between 0.5 and 1.0 microns and with an l/d ratio of 0.3.

Elemental analysis showed that the product contained a significant amount of Zn, namely 4.1 wt %. The molar composition of the product calculated from the elemental analysis was: $K_2O$/0.34 ZnO/0.77 $Al_2O_3$/4.9 $SiO_2$. It can be seen that in this composition the $K_2O/Al_2O_3$ ratio is 1.30 while the ratio $K_2O/(Al_2O_3 + ZnO)$ is 0.90. If Zn is a framework component the latter ratio should be close to unity which is the case. Hence this is another indication that zinc is a framework component.

EXAMPLE 2

Synthesis mixture with $Zn/Al_2$ atomic ratio of 1.00

In this synthesis the $Zn/Al_2$ ratio was increased from 0.50 to 1.00. A synthesis mixture with a molar composition of 3.39 $K_2O$/0.50 ZnO/0.5O $Al_2O_3$/10 $SiO_2$/163 $H_2O$ was prepared and crystallized in the same way as described in Example 1. The product yield was 12.1 wt %.

Again, XRD showed that the product was excellently crystalline and pure. The toluene adsorption capacity was 9.9 %. SEM showed that the product consisted of disk-type crystals with a diameter of about 0.4 microns and with an l/d ratio of about 0.2. The basal planes were remarkably flat and clean.

4

EP 0 543 898 B1

Elemental analysis showed that the product contained 6.0 wt % Zn. The composition of the product calculated from the elemental analysis was:

$K_2O/0.48\ ZnO/0.56\ Al_2O_3/4.5\ SiO_2$. Again the ratio $K_2O/(Al_2O_3 + ZnO)$ was close to unity, at 0.96.

It was investigated whether the product was thermally stable at 500°C. A sample of the product was heated to 510°C in air and kept at this temperature for 16 hours. XRD-crystallinity and toluene adsorption of the calcined product were identical to the as-synthesized product. From these results it was concluded that the presence of zinc did not change the thermal stability of the zeolite.

EXAMPLE 3

Synthesis mixture with $Zn/Al_2$ atomic ratio of 3.0.

A synthesis mixture with a molar composition of: $3.39\ K_2O/0.75\ ZnO/0.25\ Al_2O_3/10\ SiO_2/164\ H_2O$ was prepared as described in Example 1 and crystallized for 48 hours at 150°C. A spot sample taken from the autoclave showed that the product was completely amorphous.

After 168 hours of heating traces of zeolite-L appeared. This indicated that further increasing the amount of zinc in the synthesis mixture reduces the formation of the zeolite.

The synthesis was repeated but the alkalinity of the synthesis mixture ($K_2O/SiO_2$ ratio) was increased from 0.34 to 0.45. The composition of the synthesis mixture was: $4.50\ K_2O/0.75\ ZnO/0.25\ Al_2O_3/10\ SiO_2/165\ H_2O$. During the crystallization at 150°C spot samples were taken from the autoclave to follow the formation of the zeolite. The observations are given below:

| Time into heating at 150 °C, hours | formation of zeolite-L as observed by XRD |
|---|---|
| 53 | most intense L-peaks appeared |
| 135 | formation well underway |
| 200 | crystallinity not significantly increased vs 135 hrs ( + 19%). |

The crystallization was stopped after 200 hours. The product was washed and dried at 150°C. The product yield was 9.5 %. Again, the product yield was significantly higher than that obtained using this type of synthesis mixture in which no zinc was present (~3.5 wt %). SEM micrographs showed that the product consisted of extremely flat disk-like crystals with a diameter of about 1.0-1.5 microns and with an l/d ratio of < 0.05. The XRD diffractogram is shown in Figure 1(c).

EXAMPLE 4

Preparation of Zinc containing KL with an increased crystal length

The crystals of the zinc-containing KL-species all showed a disk-type morphology, e.g. crystals with a relatively large diameter and with a length between 0.05 and 0.1 microns. For some uses of zeolites, e.g. when using a zeolite as an aromatization catalyst, it is preferred to use crystals of a greater length. Therefore, an attempt was made to synthesize Zn-containing KL species with a more preferred morphology, e.g. an increased length while maintaining the size of the diameter and the flatness of the basal planes. The flatness of the basal planes may be measured by the ratio of maximum axial length (l) to minimum axial length (h) of a cylindrical crystal. In a cylinder with completely flat basal planes l = h and l/h = 1. Any doming of or growths on the basal planes results in h greater than l and so l/h is less than 1. The synthesis of crystals with a more preferred morphology is described below:

A synthesis mixture was prepared in which the alkalinity ($K_2O/SiO_2$ ratio) was reduced from 0.34 to 0.30. This reduces the formation rate, which can be compensated by increasing the synthesis temperature.

The $Zn/Al_2$ atomic ratio in the gel was 1.0.

The composition of the mixture was:

$3.00\ K_2O/0.50\ ZnO/0.48\ Al_2O_3/10\ SiO_2/160\ H_2O$.

This mixture was divided between two 300 ml stainless steel autoclaves and crystallized at 150°C and 175°C respectively. Both mixtures were crystallized for 66 hours. The product yields were 13.0 % for the 150°C synthesis and 13.1 % for the 175°C synthesis. Both products were excellently crystalline and pure.

5

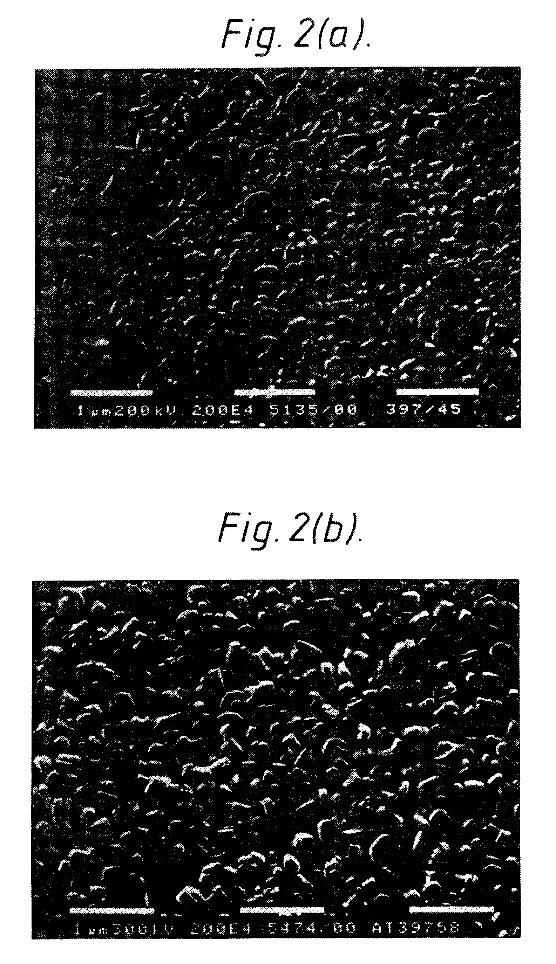

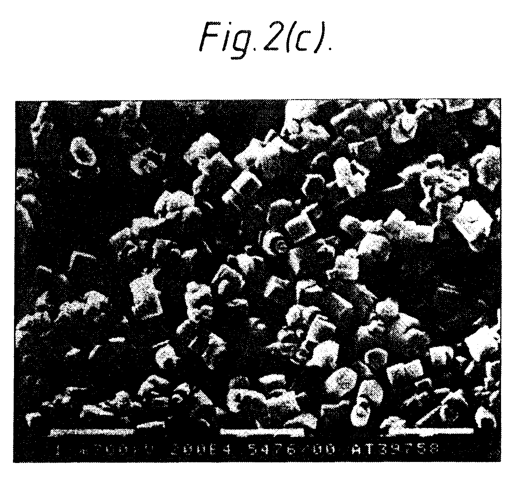

SEM micrographs showed that the length of the crystals was increased from ~0.1 micron to ~0.2 for the 150°C product and to 0.4 for the 175 °C product. Comparative SEM micrographs (magnification 20,000 times) showing the effect of the reduced alkalinity and increased crystallization temperature on the length of the crystals are shown in Figure 2.

The synthesis mixtures, crystallization temperature and approximate crystal sizes of the micrographs in Figure 2 are as follows:

| Figure | Zn/Al$_2$ atomic ratio | K$_2$O/SiO$_2$ ratio | T(°C) | crystal diam ($\mu$m) | crystal lgth ($\mu$m) |
|---|---|---|---|---|---|
| 2a | 1.0 | 0.34 | 150 | 0.4 | 0.1 |
| 2b | 1.0 | 0.30 | 150 | 0.5 | 0.2 |
| 2c | 1.0 | 0.30 | 175 | 0.5 | 0.4 |

These figures show that reducing the alkalinity or increasing the crystallization temperature increases the crystal length.

The use of the Zn-containing zeolite of Example 1 is illustrated in the following Example:

EXAMPLE 5

SELECTIVE HYDROGENATION OF BENZENE TO CYCLOHEXENE

2.5 g ZnKL of Example 1 was loaded via the incipient wetness method with Ru(NH$_3$)$_6$Cl$_3$ to obtain 3 wt % ruthenium on the catalyst. The precursor was dried overnight at 150°C. 1.0 g 3 % Ru-ZnKL was loaded in a stirred autoclave, containing 100 mL benzene and 100 mL water (or 100 mL 1 m KOH). At ambient temperature the autoclave was three times flushed with nitrogen and hydrogen. At atmospheric pressure the autoclave was heated up to 175°C.

At that temperature the total pressure was maintained at 50 bar by the addition of high pressure hydrogen. At selected intervals samples were taken and analyzed by gas chromatography.

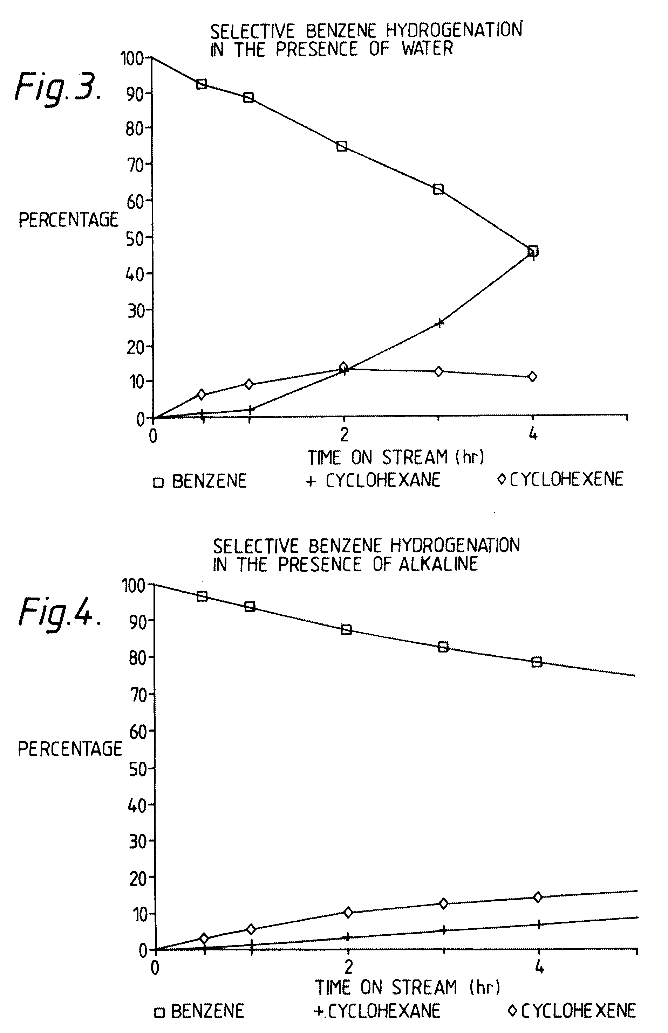

The results are shown in Tables I and II below; these data are represented graphically in Figures 3 and 4.

TABLE I

| SELECTIVE HYDROGENATION OF BENZENE IN THE PRESENCE OF WATER | | | |
|---|---|---|---|
| TIME (in hours) | BENZENE | CYCLOHEXANE | CYCLOHEXENE |
| 0 | 100 | 0 | 0 |
| 0.5 | 92.5 | 1.3 | 6.2 |
| 1 | 88.8 | 2.3 | 8.9 |
| 2 | 74.5 | 12.7 | 12.8 |
| 3 | 62.4 | 25.6 | 12.2 |
| 4 | 45.7 | 44.2 | 10.1 |

6

TABLE II

| SELECTIVE HYDROGENATION OF BENZENE IN THE PRESENCE OF ALKALINE MEDIUM | | | |
|---|---|---|---|
| TIME (in hours) | BENZENE | CYCLOHEXANE | CYCLOHEXENE |
| 0 | 100 | 0 | 0 |
| 0.5 | 96.5 | 0.6 | 2.9 |
| 1 | 93.3 | 1.3 | 5.4 |
| 2 | 87.1 | 2.8 | 10.1 |
| 3 | 82.5 | 4.9 | 12.6 |
| 4 | 78.5 | 6.9 | 14.6 |
| 5 | 74.8 | 8.8 | 16.4 |

Using the previous catalyst, (1-3 % Ru on a support, water, containing upto 4 M KOH and zinc ions, benzene, 175 °C, 50 bar hydrogen pressures) the catalyst showed upto 80 % selectivity to cyclohexene at 5-10 % benzene conversion levels after 7 hours reaction time.

Using as a catalyst 3 % Ru on the ZnKL of Example 1 gave remarkably good results:
- upto 12.8 % cyclohexene (selectivity to cyclohexene of 50%) at 25 % benzene conversion in the presence of only water.
- upto 16.4 % cyclohexene (selectivity to cyclohexene of 65 %) at 25 % benzene conversion in the presence of 1 m KOH.

High activity and selectivity to cyclohexene can be obtained with the Ruthenium on ZnKL zeolite, where zinc is part of the zeolite frame work. In the absence of alkaline in the water phase the initial selectivity to cyclohexene is high (± 75 %). When the reaction was continued, the selectivity to cyclohexene dropped to 20 % at > 50 % benzene conversion. In the presence of alkaline in the water phase the initial selectivity to cyclohexene is higher (> 80%). Even when the reaction was prolonged the selectivity remained high (> 65%). However, in the presence of alkaline the activity of the catalyst dropped with 50 % in comparison with the alkaline free reaction.

## Claims

1. A zeolite L comprising silicon, aluminium and 1 to 10% by weight of non-exchangeable zinc.

2. A zeolite as claimed in claim 1 which comprises 2 to 7% by weight of zinc.

3. A zeolite as claimed in claim 1 or 2 which is a zeolite KL.

4. A zeolite as claimed in claim 3 having the molar composition

   $K_2O/0.1\text{-}0.6\ ZnO/0.4\text{-}0.9\ Al_2O_3/4\text{-}6\ SiO_2$.

5. A zeolite as claimed in 3 or 4 in which the $K_2O/(Al_2O_3 + ZnO)$ ratio is 0.85-1.15.

6. A process for producing a zeolite as claimed in any one of the preceding claims in which a synthesis mixture comprising a source of ZnO, a source of $Al_2O_3$, a source of $SiO_2$ and water is heated at a temperature of 140 to 200 °C.

7. A process according to claim 6 in which the synthesis mixture contains L-seed crystals.

8. A process in accordance with claim 6 for producing a zeolite KL in which the synthesis mixture comprises a source of $K_2O$ and, if seed crystals are present, KL-seed crystals.

9. A process in accordance with any one of claims 6 to 8 in which the mixture is heated for at least 40 hours.

10. A process according to claim 7 in which the mixture is heated for 40 to 200 hours.

**11.** A process for aromatising an alkane comprising using as a catalyst a zeolite as claimed in any one of claims 1 to 5.

**12.** A process for selectively hydrogenating an unsaturated compound comprising using as a catalyst a zeolite as claimed in any one of claims 1 to 5.

**Patentansprüche**

**1.** Zeolith L, der Silicium, Aluminium und 1 bis 10 Gew.% nichtaustauschbares Zink umfaßt.

**2.** Zeolith nach Anspruch 1, der 2 bis 7 Gew.% Zink umfaßt.

**3.** Zeolith nach Anspruch 1 oder 2, der ein Zeolith KL ist.

**4.** Zeolith nach Anspruch 3 mit der molaren Zusammensetzung

$K_2O/0,1$ bis $0,6$ $ZnO/0,4$ bis $0,9$ $Al_2O_3/4$ bis $6$ $SiO_2$.

**5.** Zeolith nach Anspruch 3 oder 4, bei dem das Verhältnis von $K_2O$ zu $(Al_2O_3 + ZnO)$ $0,85$ bis $1,15$ beträgt.

**6.** Verfahren zur Herstellung eines Zeolith gemäß einem der vorhergehenden Ansprüche, bei dem eine Synthesemischung, die eine ZnO-Quelle, eine $Al_2O_3$-Quelle, eine $SiO_2$-Quelle und Wasser umfaßt, auf eine Temperatur von 140 bis 200 °C erhitzt wird.

**7.** Verfahren nach Anspruch 6, bei dem die Synthesemischung Zeolith L-Impfkristalle enthält.

**8.** Verfahren gemäß Anspruch 6 zur Herstellung eines Zeolith KL, bei dem die Synthesemischung außerdem eine $K_2O$-Quelle und, falls Impfkristalle vorhanden sind, KL-Impfkristalle umfaßt.

**9.** Verfahren gemäß einem der Ansprüche 6 bis 8, bei dem die Mischung mindestens 40 Stunden lang erhitzt wird.

**10.** Verfahren nach Anspruch 7, bei dem die Mischung 40 bis 200 Stunden lang erhitzt wird.

**11.** Verfahren zum Aromatisieren eines Alkans, bei dem als Katalysator ein Zeolith gemäß einem der Ansprüche 1 bis 5 verwendet wird.

**12.** Verfahren zum selektiven Hydrieren einer ungesättigten Verbindung, bei dem als Katalysator ein Zeolith gemäß einem der Ansprüche 1 bis 5 verwendet wird.

**Revendications**

**1.** Zéolite L comprenant du silicium, de l'aluminium et 1 à 10 % en poids de zinc non échangeable.

**2.** Zéolite suivant la revendication 1, qui comprend 2 à 7 % en poids de zinc.

**3.** Zéolite suivant la revendication 1 ou 2, qui est une zéolite KL.

**4.** Zéolite suivant la revendication 3, ayant la composition molaire

$K_2O/0,1-0,6$ $ZnO/0,4-0,9$ $Al_2O_3/4-6$ $SiO_2$.

**5.** Zéolite suivant la revendication 3 ou 4, dans laquelle le rapport $K_2O/(Al_2O_3 + ZnO)$ a une valeur de 0,85 à 1,15.

**6.** Procédé de production d'une zéolite suivant l'une quelconque des revendications précédentes, dans lequel un mélange de synthèse comprenant une source de ZnO, une source de $Al_2O_3$, une source de

8

$SiO_2$ et de l'eau est chauffé à une température de 140 à 200°C.

7. Procédé suivant la revendication 6, dans lequel le mélange de synthèse contient des germes cristallins de zéolite L.

8. Procédé suivant la revendication 6 pour la production d'une zéolite KL, dans lequel le mélange de synthèse comprend une source de $K_2O$ et, si des germes cristallins sont présents, des germes cristallins de zéolite KL.

9. Procédé suivant l'une quelconque des revendications 6 à 8, dans lequel le mélange est chauffé pendant au moins 40 heures.

10. Procédé suivant la revendication 7, dans lequel le mélange est chauffé pendant 40 à 200 heures.

11. Procédé d'aromatisation d'un alcane, comprenant l'utilisation, comme catalyseur, d'une zéolite suivant l'une quelconque des revendications 1 à 5.

12. Procédé d'hydrogénation sélective d'un composé insaturé, comprenant l'utilisation, comme catalyseur, d'une zéolite suivant l'une quelconque des revendications 1 à 5.

Fig.1.

(a) CYLINDRICAL ALUMINA KL

(b) Zn-CONTAINING KL OF EXAMPLE 1

(c) ZINC-CONTAINING KL, OF EXAMPLE 3.

## Fig. 2(a).

## Fig. 2(b).

*Fig.2(c).*

**Fig.3.**

SELECTIVE BENZENE HYDROGENATION IN THE PRESENCE OF WATER

PERCENTAGE vs TIME ON STREAM (hr)

□ BENZENE    + CYCLOHEXANE    ◇ CYCLOHEXENE

**Fig.4.**

SELECTIVE BENZENE HYDROGENATION IN THE PRESENCE OF ALKALINE

PERCENTAGE vs TIME ON STREAM (hr)

□ BENZENE    +.CYCLOHEXANE    ◇ CYCLOHEXENE

13